# EUROPEAN PATENT SPECIFICATION

(11) **EP 3 355 758 B1**
(45) Date of publication and mention of the grant of the patent: **26.07.2023**
(21) Application number: 16852710.9
(22) Date of filing: 30.09.2016
(51) Int. Cl.: A61B 1/00, A61B 1/04, A61B 1/06, A61B 1/07

(54) **TETHERED OPTICAL IMAGING PROBE WITH A CAPSULE ENCLOSING A MOTOR AND A REFLECTIVE SURFACE ROTATABLY COUPLED TO THE MOTOR**
SONDE ZUR OPTISCHEN BILDGEBUNG, AN EINEM KABEL GEBUNDEN UND MIT EINER EINEN MOTOR UND EINE AN DEM MOTOR DREHBAR GEKOPPELTE REFLEKTIERENDE FLÄCHE UMGEBENDEN KAPSEL
SONDE D'IMAGERIE OPTIQUE, ATTACHÉE À UN CÂBLE, AVEC UNE CAPSULE ENVELOPPANT UN MOTEUR ET UNE SURFACE RÉFLÉCHISSANTE COUPLÉE DE MANIÈRE ROTATIVE AU MOTEUR

(30) Priority: 30.09.2015 US 201562235175 P
(43) Date of publication of application: 08.08.2018
(73) Proprietor: The General Hospital Corporation, Boston, MA 02114 (US)
(72) Inventor: TEARNEY, Guillermo J., Boston, MA 02114 (US); REDDY, Rohith K., Houston, TX 77003 (US); GORA, Michalina J., Boston, MA 02114 (US); CHU, Kengyeh K., Jamaica Plain, MA 02130 (US); BEATTY, Matthew, Boston, MA 02114 (US)
(74) Representative: Maiwald GmbH
(86) International application number: PCT/US2016/054772
(87) International publication number: WO 2017/059246

(56) References cited:
- WO-A1-2013/177154
- WO-A1-2013/177154
- WO-A1-2015/020124
- WO-A1-2015/072432
- WO-A2-2009/102445
- WO-A2-2014/033111
- CN-U- 204 204 473
- US-A- 3 629 677
- US-A- 5 193 526
- US-A- 5 286 922
- US-A1- 2003 067 227
- US-A1- 2005 143 644
- US-A1- 2007 299 309
- US-A1- 2008 262 312
- US-A1- 2013 063 964
- US-A1- 2013 345 510
- US-A1- 2014 155 753

## Description

### CROSS-REFERENCES TO RELATED APPLICATIONS

The present disclosure is based on and claims priority to United States Provisional Patent Application No. 62/235,175, filed September 30, 2015, and entitled "Actively Controlled Optical Imaging Device."

### STATEMENT REGARDING FEDERALLY SPONSORED RESEARCH

Not Applicable.

### BACKGROUND

The disclosure relates generally to optical imaging systems and, more specifically, to a tethered optical imaging probe for medical diagnosis.

Tethered optical imaging devices are used in some medical imaging applications in an attempt to replace more invasive medical procedures. For example, diagnosis of diseases in the gastrointestinal (GI) tract, which are typically diagnosed by an endoscopy where a patient is anesthetized, may be carried out via a tethered optical imaging device that is swallowed by a patient.

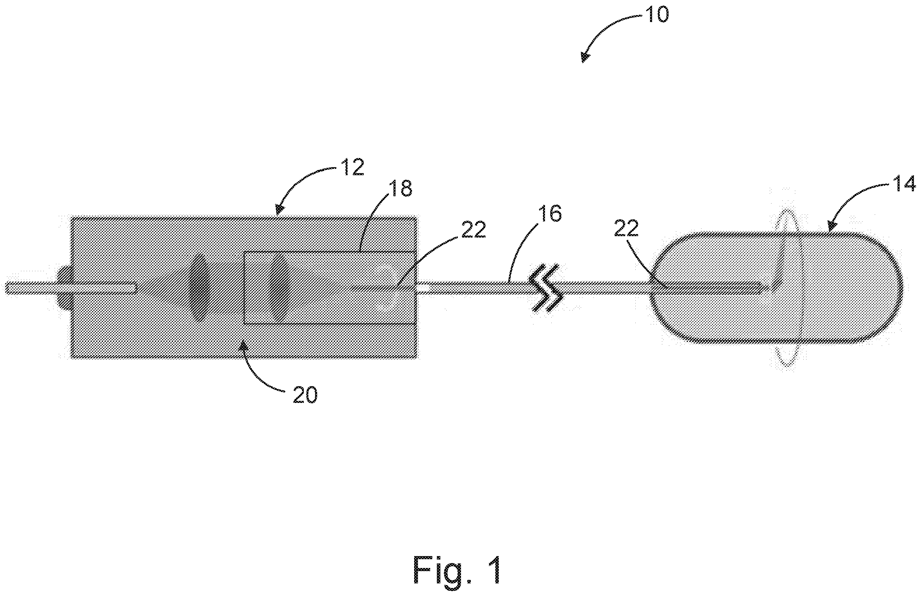

A current tethered optical imaging device 10 is illustrated in Fig. 1. As shown in Fig. 1, the tethered optical imaging device 10 includes a rotary junction 12 coupled to a capsule 14 by a sheath 16. The rotary junction 12 is a fixed component of the optical imaging device 10 (i.e., non-disposable and required) that presents a significant cost (e.g., ~$10,000). The significant cost of the rotary junction 12 results from the complexity of the component. That is, the rotary junction 12 includes a motor 18 and a plurality of optics 20 arranged within the rotary junction 12. Some of the plurality of optics 20 are rotatably coupled to the motor 18 for rotation therewith. The motor 18 is also rotatably coupled to an optical fiber 22 that is arranged within the sheath 16 and extends into the capsule 14.

The design of the current tethered optical imaging device 10 leads to various complexities and deficiencies associated with the device. For example, some of the plurality of optics 20 are required to rotate within the rotary junction 12, which requires additional components to facilitate transmitting light therethrough. The sheath 16 is required to define a larger diameter in order to accommodate the rotating optical fiber 22 therein. The mechanical properties of the sheath 16 and optical fiber 22 must provide a low friction between the two components to ensure proper operation. Additionally, the optical properties (e.g., transmission efficiency, beam profile) of the rotating optical fiber 22 may change as the sheath is bent during operation. The document WO 2013/177154 A1 discloses a tethered optical imaging probe with a capsule enclosing a motor and a reflective surface.

Due to the deficiencies in current tethered optical imaging devices, it would be desirable to have a tethered optical imaging probe that is easily integrated into an optical system and that is fabricated entirely of low-cost, disposable components.

### BRIEF SUMMARY

The present disclosure provides a tethered optical imaging probe configured to be integrated into an optical system for medical diagnosis. In one configuration, the present disclosure provides a tethered optical imaging probe including a motor arranged within a swallowable capsule. A rotational speed of the motor is actively controlled by a feedback signal.

In one aspect, the present disclosure provides a tethered optical imaging probe including a motor, a reflective surface rotatably coupled to the motor, and a capsule enclosing the motor and the reflective surface. The capsule is dimensioned to be swallowable by a patient. The tethered optical imaging probe further includes a tether coupled to a first end of the capsule. The tether includes an optical waveguide arranged therein. The optical waveguide is arranged to receive source light at a distal end of the optical waveguide and project the source light from a proximal end of the optical waveguide onto the reflective surface and/or to receive reflected light from the reflective surface at the proximal end of the optical waveguide and transmit the reflected light to the distal end of the optical waveguide.

In one aspect, the present disclosure provides a tethered capsule endomicroscopy system including an optical source arranged to emit source light, and an optical imaging probe. The optical imaging probe includes a motor, a reflective surface rotatably coupled to the motor, and a capsule enclosing the motor and the reflective surface. The capsule is dimensioned to be swallowable by a patient. The optical imaging probe further includes a tether coupled to a first end of the capsule. The tether includes an optical waveguide arranged therein. The optical waveguide is arranged to receive the source light at a distal end of the optical waveguide and project the source light from a proximal end of the optical waveguide onto the reflective surface and/or to receive reflected light from the reflective surface at the proximal end of the optical waveguide and transmit the reflected light to the distal end of the optical waveguide. The tethered capsule endomicroscopy system further includes an optical detector arranged to detect the reflected light from the reflective surface transmitted to the distal end of the optical waveguide, and a controller configured to reconstruct the reflected light detected by the optical detector into cross-sectional morphological data.

In one aspect, the present disclosure provides an optical imaging system including a tethered optical imaging probe. The tethered optical imaging probe includes a motor coupled to an optical component and configured to selectively rotate the optical component at a rotational speed, and a capsule enclosing the motor and the optical component. The capsule is dimensioned to be swallowable by a patient. The tethered optical imaging probe further includes a detection object arranged within the capsule, and a tether coupled to a first end of the capsule. The tether includes an optical waveguide arranged therein. A signal is generated as the optical component is rotatably positioned by the motor to direct emitted light from the detection object to the optical waveguide. The optical imaging system further includes a controller in communication with the motor and configured to control the rotational speed of the motor to a desired rotational speed based on feedback from the signal.

In one aspect, the present disclosure provides an optical imaging system including an optical source arranged to emit source light, and an optical imaging probe. The optical imaging probe includes a motor, a reflective surface rotatably coupled to the motor, and a capsule enclosing the motor and the reflective surface. The capsule is dimensioned to be swallowable by a patient. The optical imaging probe further includes a detection object arranged within the capsule, and a tether coupled to a first end of the capsule. The tether includes an optical waveguide arranged therein. The optical waveguide is arranged to receive the source light at a distal end of the optical waveguide and project the source light from a proximal end of the optical waveguide onto the reflective surface and/or to receive reflected light from the reflective surface at the proximal end of the optical waveguide and transmit the reflected light to the distal end of the optical waveguide. A signal is generated as the reflective surface is rotatably positioned by the motor to direct emitted light from the detection object to the proximal end of the optical waveguide for transmission to the distal end of the optical waveguide. The optical imaging system further includes an optical detector arranged to detect the reflected light from the reflective surface transmitted to the distal end of the optical waveguide, and a controller in communication with the motor and configured to reconstruct the reflected light detected by the detector into cross-sectional morphological data. The controller is further configured to control a rotational speed of the motor to a desired rotational speed based on feedback from the signal.

In one aspect, the present disclosure provides a method for controlling a tethered optical imaging probe. The tethered optical imaging probe includes a motor, a reflective surface rotatably coupled to the motor, a capsule enclosing the motor and the reflective surface, and a tether coupled to a first end of the capsule and having an optical waveguide arranged therein. The tethered optical imaging probe further includes a detection object arranged within the capsule. The capsule is dimensioned to be swallowable by a patient. The optical waveguide is arranged to receive source light at a distal end of the optical waveguide and project the source light from a proximal end of the optical waveguide onto the reflective surface and/or to receive reflected light from the reflective surface at the proximal end of the optical waveguide and transmit the reflected light to the distal end of the optical waveguide. The method includes outputting source light from the optical waveguide onto the reflective surface, and rotating the reflective surface, via the motor, thereby forming a circumferential optical path formed by the source light output from the optical waveguide onto the reflective surface as the reflective surface is rotated. The detection object is arranged in the circumferential optical path. The method further includes generating a signal each time the reflective surface directs light emitted from the detection object to the proximal end of the optical waveguide, and controlling a rotational speed of the motor using the generated signal.

In one aspect, the present disclosure provides a method for controlling a tethered optical imaging probe. The tethered optical imaging probe includes a motor, a reflective surface rotatably coupled to the motor, a capsule enclosing the motor and the reflective surface, and a tether coupled to a first end of the capsule and having an optical waveguide arranged therein. The tethered optical imaging probe further includes a light emitting object arranged within the capsule. The capsule is dimensioned to be swallowable by a patient. The optical waveguide is arranged to receive reflected light from the reflective surface at the proximal end of the optical waveguide and transmit the reflected light to the distal end of the optical waveguide. The method includes rotating the reflective surface, via the motor, thereby forming a circumferential field of view in which emitted light is periodically directed to the reflective surface and reflected to the proximal end of the optical waveguide as the reflective surface is rotated. The light emitting object is arranged in the circumferential field of view. The method further includes generating a signal each time the reflective surface directs light emitted from the light emitting object to the proximal end of the optical waveguide, and controlling a rotational speed of the motor using the generated signal.

The foregoing and other aspects and advantages of the probe will appear from the following description. In the description, reference is made to the accompanying drawings which form a part hereof, and in which there is shown by way of illustration a preferred embodiment of the probe.

### BRIEF DESCRIPTION OF DRAWINGS

Features, aspects and advantages other than those set forth above will become apparent when consideration is given to the following detailed description thereof. Such detailed description makes reference to the following drawings.
Fig. 1 is a schematic illustration of a tethered optical imaging device according to the prior art.
Fig. 2 is a schematic illustration of an optical imaging system according to one aspect of the present disclosure.
Fig. 3 is a schematic illustration of a wireless tethered optical imaging probe of the optical system of Fig. 2 according to one aspect of the present disclosure.
Fig. 4 is a schematic illustration of a wired tethered optical imaging probe of the optical system of Fig. 2 according to another aspect of the present disclosure.
Fig. 5 is a schematic illustration of the wired tethered optical imaging probe of Fig. 4 including one or more supports arranged within a capsule.
Fig. 6 illustrates a control signal applied to either the wireless tethered optical imaging probe of Fig. 3 or the wired optical imaging probe of Fig. 4 according to one aspect of the present disclosure.
Fig. 7 is a schematic illustration of a motor control feedback strategy utilizing one or more wires according to one aspect of the present disclosure.
Fig. 8 is a schematic illustration of a motor control feedback strategy utilizing a light emitting material according to one aspect of the present disclosure.
Fig. 9 is a schematic illustration of a motor control feedback strategy utilizing a secondary optical waveguide according to one aspect of the present disclosure.
Fig. 10 is graph illustrating a motor speed as a function of time with a constant voltage applied to the motor.
Fig. 11 is a graph illustrating a motor speed as a function of time with a constant voltage applied to the motor, a periodic control signal applied to the motor, and a periodic control signal applied to the motor with a feedback signal.
Fig. 12 is a graph illustrating a deviation profile as a function of time of the motor speed of Fig. 11 with a constant voltage applied to the motor.
Fig. 13 is a graph illustrating a deviation profile as a function of time of the motor speed of Fig 11 with a periodic voltage applied to the motor.
Fig. 14 is a graph illustrating a deviation profile as a function of time of the motor speed of Fig. 11 with a periodic voltage applied to the motor with a feedback signal.
Fig. 15 is a graph illustrating a position error as a function of time for a motor with a constant voltage applied to the motor, a periodic control signal applied to the motor, and a periodic control signal applied to the motor with a feedback signal.
Fig 16 is a graph illustrating motor speed as a function of time at a motor speed of 20 Hz and 40 Hz with and without a damping weight coupled to the motor.

### DETAILED DESCRIPTION

Before the present probe is described in further detail, it is to be understood that the probe is not limited to the particular embodiments described. It is also to be understood that the terminology used herein is for the purpose of describing particular embodiments only. The invention is defined by the appended claims.

Numeric ranges disclosed herein are inclusive of their endpoints. For example, a numeric range of between 1 and 10 includes the values 1 and 10. When a series of numeric ranges are disclosed for a given value, the present disclosure expressly contemplates ranges including all combinations of the upper and lower bounds of those ranges. For example, a numeric range of between 1 and 10 or between 2 and 9 is intended to include the numeric ranges of between 1 and 9 and between 2 and 10.

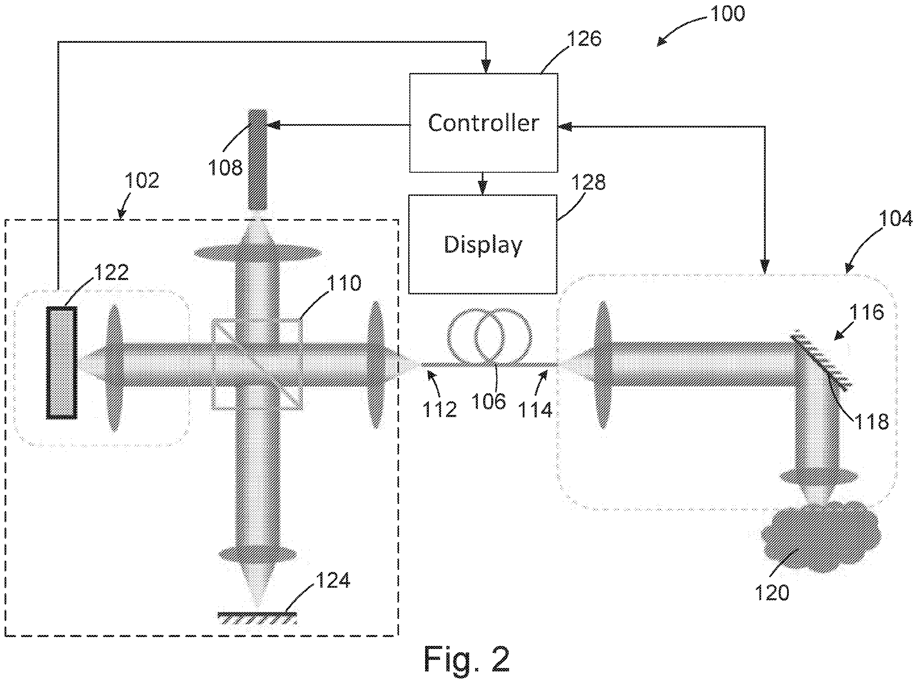

Fig. 2 illustrates one example of an optical system 100 according to the present disclosure. The optical system 100 includes an external optical assembly 102 optically coupled to a tethered optical imaging probe 104 via an optical waveguide 106. The external optical assembly 102 is configured to receive source light from a light source 108, and includes a beam splitter 110 to reflect the source light into a distal end 112 of the optical waveguide 106. In some examples, the light source 108 may be in the form of a broadband laser configured to sweep wavelengths in the near-infrared spectrum.

The optical waveguide 106 is configured to efficiently transmit the source light along the optical waveguide 106 from the distal end 112 to a proximal end 114 of the optical waveguide 106. The proximal end 114 of the optical waveguide 106 is coupled to the tethered optical imaging probe 104 and arranged to project the source light from the proximal end 114 onto an optical component 116 within the tethered optical imaging probe 104.

The illustrated optical component 116 is in the form of a reflective surface 118 configured to efficiently reflect the source light emitted from the proximal end 114 of the optical waveguide 106 onto a sample 120 (e.g., a portion of a luminal organ or tissue within a patient). The proximal end 114 of the optical waveguide 106 is arranged to receive reflected light from the sample 120 off of the reflective surface 118. The reflected light is transmitted along the optical waveguide 106 from the proximal end 114 to the distal end 112. In some examples, the optical waveguide 106 may be in the form of an optical fiber, an optical fiber bundle, a plurality of optical fibers, or other structures known by those having ordinary skill in the optical arts to be suitable for use as an optical waveguide 106. It should be appreciated that the optical waveguide 106 is not limited to a single optical fiber that performs both transmission of the source light and the reflected light from the sample 120. That is, in some examples, the optical waveguide 106 may comprise a fiber bundle where a core is arranged to transmit one of the source light or the reflected light from the sample 120 and surrounding fibers are arranged to transmit the other of the source light or the reflected light from the sample 120. In other examples, the optical waveguide 106 may comprise two totally separate fibers, one for transmitting source light to the reflective surface 118 and another for transmitting reflected light from the sample 120.

The reflected light from the sample 120 transmitted to the distal end 112 of the optical waveguide 106 transmits through the beam splitter 110 to an optical detector 122. In some examples, the optical detector 122 may be in the form of a CCD array, a CMOS array, photodiodes (made of silicon, germanium, InGaAs, lead sulfide, or other materials), photocells, photoresistors, phototransistors or other photosensors. A portion of the source light output by the light source 108 transmits through the beam splitter 110 to a reference mirror 124. The portion of the source light incident on the reference mirror 124 reflects off the reference mirror 124 and is directed by the beam splitter 110 to the optical detector 122. Thus, in operation, the optical detector 122 receives both reference source light reflected from the reference mirror 124 and reflected light off the reflective surface 118 from the sample 120.

A controller 126 is in communication with the tethered optical imaging probe 104, the light source 108, the optical detector 122, and a display 128. The controller 126 may be in wired communication (e.g., via Ethernet, USB, CAN) with the tethered optical imaging probe 104, the light source 108, the optical detector 122, and/or the display 128. Alternatively or additionally, the controller 126 may be in wireless communication (e.g., via Bluetooth^{®}, WiFi) with the tethered optical imaging probe 104, the light source 108, the optical detector 122, and/or the display 128.

The controller 126 is configured to control the operation of the tethered optical imaging probe 104, as will be described below. The controller 126 is further configured to reconstruct the reflected light off the reflective surface 118 from the sample 120 that is detected by the optical detector 122 into cross-sectional morphological data. In one example, the optical system 100 is configured to implement an optical frequency domain imaging technique to generate the cross-sectional morphological data. Sequential cross-sections may also be compiled to reconstruct a three-dimensional representation, for example, of an entire luminal organ of a patient.

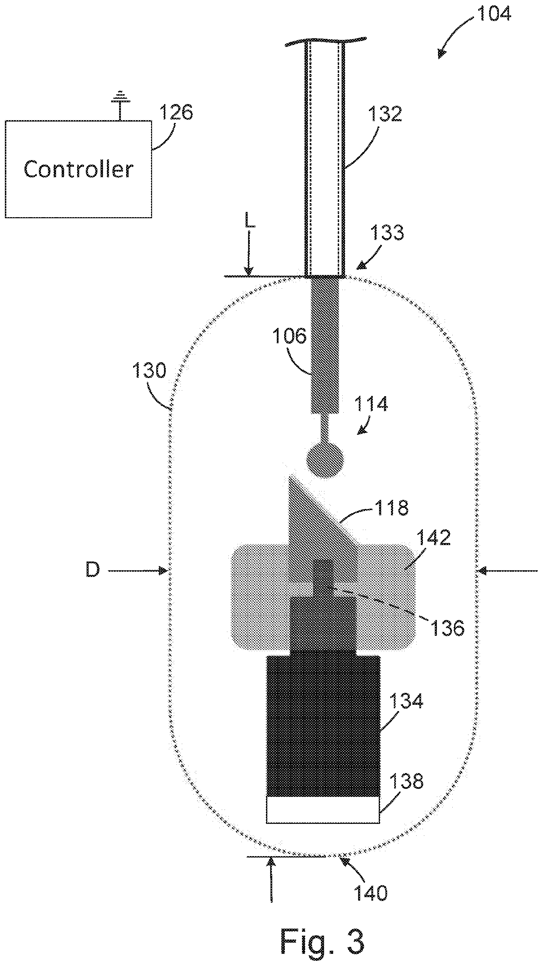

Fig. 3 illustrates one example of the tethered optical imaging probe 104 according to the present disclosure. The tethered optical imaging probe 104 includes a capsule 130 and a tether 132 coupled to a first end 133 of the capsule 130. The capsule 130 is dimensioned to be swallowable by a patient, which can be in certain cases, a human patient such as an adult or a child patient, and which can be in other cases, a veterinary patient. The capsule 130 defines a generally cylindrical shape with hemispherical ends. The capsule 130 defines a capsule diameter D and a capsule length L. In some examples, the capsule diameter D may be between approximately 5 millimeters (mm) and approximately 20 mm. In other examples, the capsule diameter D may be between approximately 10 mm and approximately 15 mm. In some examples, the capsule length L may be between approximately 20 mm and approximately 30 mm. In other examples, the capsule length L may be between approximately 22 mm and approximately 28 mm.

The capsule 130 is fabricated from a biocompatible material configured to efficiently transmit the source light reflected from the reflective surface 118 through the capsule 130 onto the sample 120 and to efficiently transmit the reflected light from the sample 120 through the capsule 130 onto the reflective surface 118. In some examples, the capsule 130 may be fabricated from PMMA in combination with other plastics or metals like stainless steel or brass.

The tether 132 includes the optical waveguide 106 arranged therein. As will be described, the design tethered optical imaging probe 104 negates the need to rotate the optical waveguide 106. The tether 132 may define a substantially reduced diameter when compared to the sheath 16 of the prior art device 10, and does not need to be fabricated from low-friction materials to compensate for a rotating optical fiber. Thus, the tether 132 provides better flexibility and a substantially reduced cost (e.g., ~$0.01 compared to ~$200) when compared to the sheath 16 of the prior art device 10. The tether 132 is fabricated from a biocompatible material (e.g., polyimide, PEBAX^{®}, PTFE, FEP).

A motor 134 and the reflective surface 118 are enclosed within the capsule 130. The motor 134 includes a drive shaft 136 that rotatably couples the reflective surface 118 to the motor 134. In operation, as the motor 134 rotates the drive shaft 136, the reflective surface 118 rotates with the drive shaft 136. In the illustrated example, the motor 134 is powered by a power supply 138 arranged within the capsule 130. The power supply 138 may be in the form of a battery, a rechargeable battery, a solar cell. In the illustrated example, the controller 126 is configured to wirelessly communicate with the motor 134. As will be described, the controller 126 is configured to supply a periodic control signal to the motor 134 to control a rotational speed of the motor.

The motor 134 is arranged within the capsule 130 such that the drive shaft 136 extends toward the first end 133 of the capsule 130, thereby arranging the reflective surface 118 rotatably coupled thereto adjacent to the proximal end 114 of the optical waveguide 106. In other examples, the motor 134 may be rearranged to extend toward a second end 140 of the capsule opposite the first end 133. That is, the directional orientation of the motor 134, the proximal end 114 of the optical waveguide 106, and the reflective surface 118 within the capsule 130 may be arranged differently as would be appreciated by one of skill in the art.

The motor 134 utilized in the tethered optical imaging probe 104 is disposable and low-cost (e.g., between ~$1 and $10). In one example, the motor 134 may be a cell phone motor typically used to generate vibrations in a cell phone. Low-cost motors are generally incapable of being utilized in high precision imaging applications; however, the present disclosure provides an approach to accurately and precisely control the motor 134 to facilitate the acquisition of high quality imaging data, as will be described. Due to the low cost of the components used to manufacture the tethered optical imaging probe 104, the tethered optical imaging probe 104 may entirely disposable after use without an end user sustaining significant costs.

The drive shaft 136 of the motor 134 may include a damping weight 142 coupled thereto for rotation therewith. As will be described, the damping weight 142 reduces short-term fluctuations in a rotational speed of the motor 134 by increasing a moment of inertia. The damping weight 142 defines a generally cylindrical shape. The damping weight 142 may be fabricated from a metal material (e.g., brass). In some examples, the damping weight 142 may define a weight of between approximately 0.01 grams (g) and 4 g.

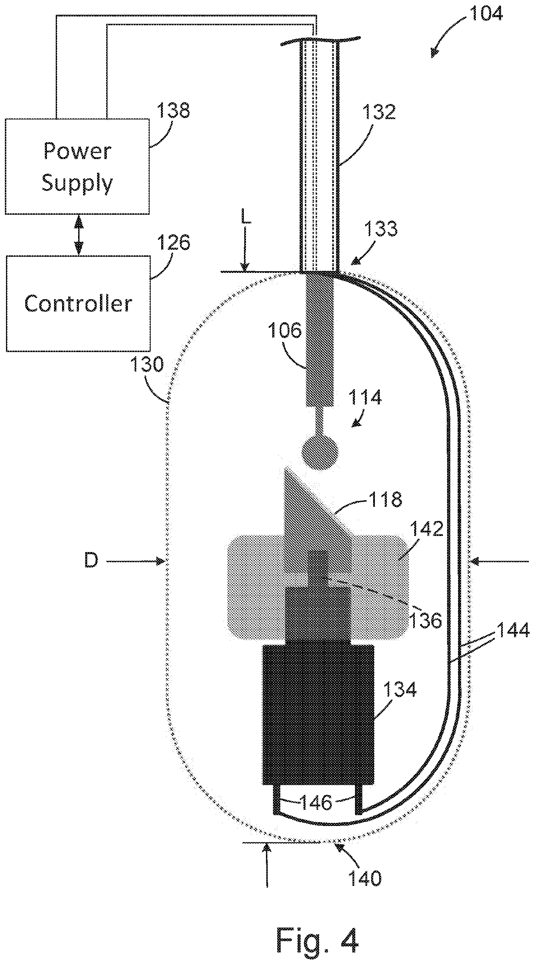

Fig. 4 illustrates another example of the tethered optical imaging probe 104 according to the present disclosure. The tethered optical imaging probe 104 of Fig. 4 is similar to the tethered optical imaging probe 104 of Fig. 3, except as described below or as shown in the figures. Similar components are identified using like reference numerals. As shown in Fig. 4, the tethered optical imaging probe 104 includes one or more wires 144 extending into and along the capsule 130. Each of the wires 144 is coupled to a corresponding terminal 146 of the motor 134. The wires 144 are arranged within the tether 132 and extend to the power supply 138.

In the illustrated example, the power supply 138 is arranged externally from the capsule 130. The power supply 138 is in communication with the controller 126. In this example, the controller 126 may be in wired or wireless communication with the power supply 138. In other examples, the controller 126 may include an integrated power supply, in which case, the power supply 138 may not be required.

As will be described, in one example, the wires 144 may be implemented in a control feedback strategy to control a rotational speed of the motor 134. In order to facilitate such a control feedback strategy, a circumferential position of the wires 144 within the capsule 130 may be fixed. In addition, to ensure proper optical coupling between the reflective surface 118 and the proximal end 114 of the optical waveguide 106, the motor 134 and the proximal end 114 of the optical waveguide 106 may be fixed within the capsule 130.

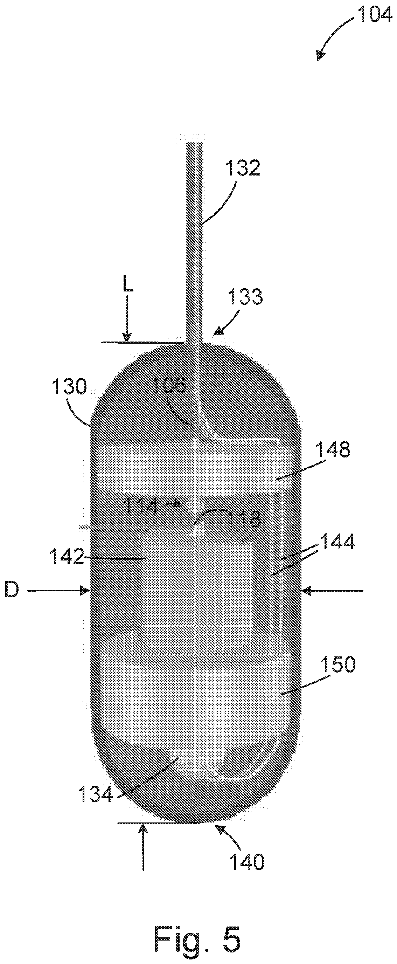

Fig. 5 illustrates one example of a mechanism for securing the proximal end 114 of the optical waveguide 106, the motor 134, and the wires 144 within the capsule 130. As shown in Fig. 5, the tethered optical imaging probe 104 may include a waveguide support 148 and a motor support 150 arranged within the capsule 130. The waveguide support 148 is secured within the capsule 130 and the proximal end 114 of the optical waveguide 106 extends therethrough to secure the proximal end 114 of the optical waveguide 106 in a fixed position adjacent to the reflective surface 118. The wires 144 extend through and are secured within a periphery of the waveguide support 148. The motor support 150 is secured within the capsule 130 and a portion of the motor 134 extends therethrough to secure the reflective surface 118 in a fixed position adjacent to the proximal end 114 of the optical waveguide 106. The wires 144 extend through and are secured within a periphery of the motor support 150. With the wires 144 extending through and secured within each of the waveguide support 148 and the motor support 150, a circumferential position of the wires 144 within the capsule 130 is fixed.

In other examples, any securing mechanism (e.g., an adhesive) may be utilized to secure the proximal end 114 of the optical waveguide 106 and/or the motor 134 within the capsule 130.

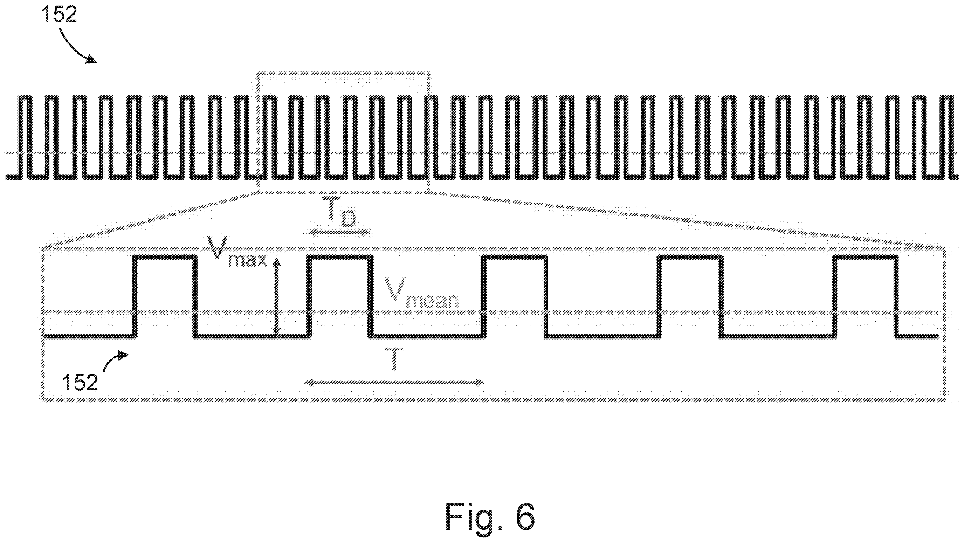

As described above, a periodic signal may be supplied to the motor 134 by the controller 126. Fig. 6 illustrates one example of a periodic control signal 152 configured to be supplied to the motor 134. As will be described, due to the inexpensive nature of the motor 134, supplying a constant voltage to the motor 134 is not sufficient to produce a controllable rotational speed suitable for high precision imaging. The use of a periodic control signal facilitates substantially increased accuracy and precision when controlling the rotational speed of the motor 134.

The illustrated periodic control signal 152 is in the form of a square wave comprising a plurality of pluses. Each of the plurality of pulses define a pulse width T_{D} and a voltage magnitude Vₘₐₓ. In some examples, the voltage magnitude Vₘₐₓ may be between approximately 0 volts (V) and 10 V. A time between subsequent pulses, or period, T is defined to be larger than a natural time constant of the motor 134. In one example, the natural time constant of the motor 134 may be an inductive-resistive time constant. In some examples, the frequency defined by the periodic control signal 152 may be greater than approximately 50 hertz (Hz) or greater than approximately 2 kilohertz (kHz). In certain cases, the frequency defined by the periodic control signal 152 can be between 50 Hz and 2 kHz.

The periodic control signal 152 defines a mean voltage Vₘₑₐₙ that is defined by a ratio of the pulse width T_{D} to the period T. The mean voltage Vₘₑₐₙ is proportional to a rotational speed of the motor 134. Thus, the pulse width T_{D} and/or the frequency defined by the periodic control signal 152 may be modulated to control a rotational speed of the motor 134.

To further facilitate accurate and precise control of the rotational speed of the motor 134 a feedback signal may be supplied to the controller 126 to enable the controller 126 to actively control the rotational speed of the motor 134 to a desired rotational speed. The feedback signal may be generated using software (e.g., via image processing) or using hardware (e.g., a signal generated within the capsule 130 and detected by the proximal end 114 of the optical waveguide 106).

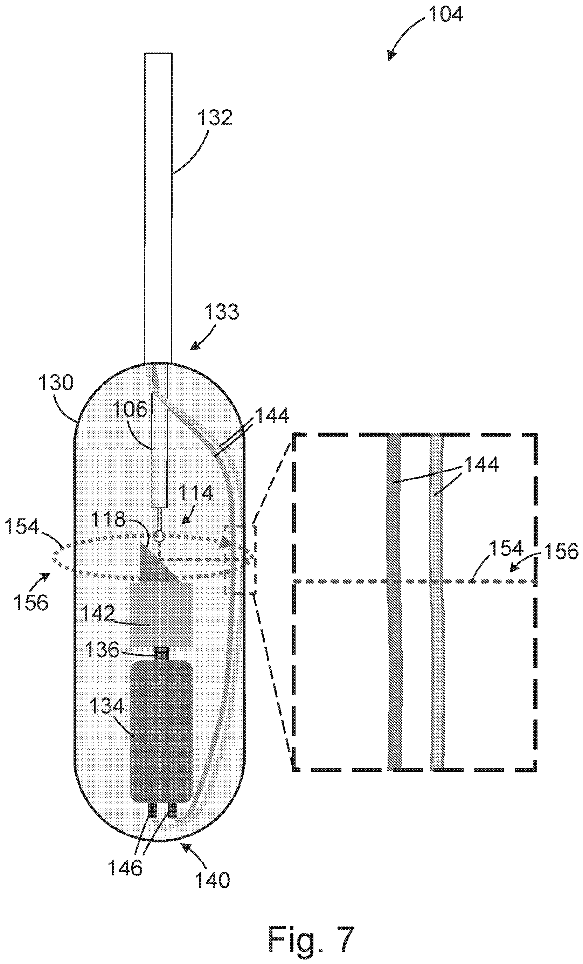

Fig. 7 illustrates one configuration for generating a feedback signal. As shown in Fig. 7, during operation, the source light is emitted from the proximal end 114 of the optical waveguide 106 onto the reflective surface 118. The source light is then reflected, at an approximately 45° degree angle, radially outward through the capsule 130 and onto the sample 120. In certain cases, the angle of reflection can be adjusted slightly from the 45° angle in order to reduce and/or eliminate back reflections from the capsule 130. In certain cases, the angle of reflection can be 41° or 49°, though other angles of reflection are contemplated. As the motor 134 rotates the reflective surface 118, the source light is rotated circumferentially around the capsule to form a circumferential optical path 154 traversed by the source light. The motor 134, the reflective surface 118, and the proximal end 114 of the optical waveguide 106 are positioned to provide a circumferential field of view 156 that encompasses a portion of the capsule 130 for detecting light reflected from the sample 120. The circumferential field of view 156 generally overlaps with the circumferential optical path 154 traversed by the source light. In this way, the reflective surface 118 is arranged to reflect source light from the proximal end 114 of the optical waveguide 106 onto the sample 120 and reflect light from the sample 120 back to the proximal end 114 of the optical waveguide 106.

In some examples, a detection object may be arranged in the circumferential optical path and/or the circumferential field of view to facilitate the generation of the feedback signal. A feedback signal generated by or from the detection object may be detected as the reflective surface 118 is rotatably positioned by the motor 134 to align with the detection object to direct the feedback signal from the detection object to the proximal end 114 of the optical waveguide 106 for transmission to the distal end 112 of the optical waveguide 106.

In the example of Fig. 7, the detection object may be in the form of the wires 144 that may be used to generate the feedback signal supplied to the controller 126. The wires 144 possess various properties that are useful in generating the feedback signal. For example, the wires 144 may cast a shadow radially in an image acquired by the optical detector 122, when the reflective surface 118 is rotatably positioned by the motor 134 to align with the wires 144. The capsule 130 may be visible in the image acquired by the optical detector 122, except where the shadow is cast by the wires 144. Since the wires 144 define a known thickness and are circumferentially fixed within the capsule 130, a change in the radial position of the shadow cast by the wires 144 in images acquired by the optical detector 122 may be utilized to generate a feedback control signal. That is, the signal from the wires 144 must occur once per rotation of the motor 134 and periodically, if the motor 134 rotates at a substantially constant speed. Any deviation of the periodicity in the signal from the wires 144 indicates a change in the rotational speed of the motor 134. This change in periodicity may be utilized to estimate a change in the rotational speed of the motor 134 and provide a feedback signal to the controller 126.

In some examples, an image processing algorithm may be implemented to detect changes in the radial position of the shadow cast by the wires 144 in the images acquired by the optical detector 122. In one example, an image processing algorithm may involve determining averages along the radial direction in the images acquired by the optical detector 122 where the wires 144 are expected to be present and regions where the capsule 130 is expected to be present. A ratio or difference of the two radial region averages may be one possible metric utilized to generate a feedback control signal. It should be appreciated that the techniques described herein utilizing the wires 144 to generate a feedback signal may only require at least one of the wires 144 to facilitate generating the feedback signal.

In some examples, the wires 144 may be coated with a material (e.g., a paint, nail polish) to enhance back-scattering of reflected source light from the wires 144 to the reflective surface 118. That is, the wires 144 may be at least partially reflective to the source light. The back-scattered, reflected source light, or signal, from the wires 144 may be identified via image processing, as described above, or via hardware detection (e.g., optical filtering and detection by reflection off of the reflective surface 118 into the proximal end 114 of the optical waveguide 106). Again, any deviation in the periodicity in the signal from the wires 144 indicates a change in the rotational speed of the motor 134 and may be utilized to provide a feedback signal to the controller 126.

In some examples, the wires 144 may be coated with a phosphorescent coating, a fluorescent coating, a quantum dot coating, or a combination thereof. In this example, the coating applied to the wires 144 may be configured to absorb a portion of the source light reflected from reflective surface 118 and subsequently emit signal light at a wavelength different than the source light back to the reflective surface 118 and into the proximal end 114 of the optical waveguide 106. The signal light emitted by the wires 144 may be optically filtered (e.g., via a wavelength division multiplexer, a dichroic mirror) and utilized to provide a feedback control signal to the controller 126. The signal light emitted from the wires 144 may be detected as the reflective surface 118 is rotatably positioned by the motor 134 to direct the emitted signal light from the light emitting material to the proximal end 114 of the optical waveguide 106. Again, any deviation in the periodicity in the signal light emitted from the wires 144 indicates a change in the rotational speed of the motor 134 and may be utilized to provide a feedback signal to the controller 126.

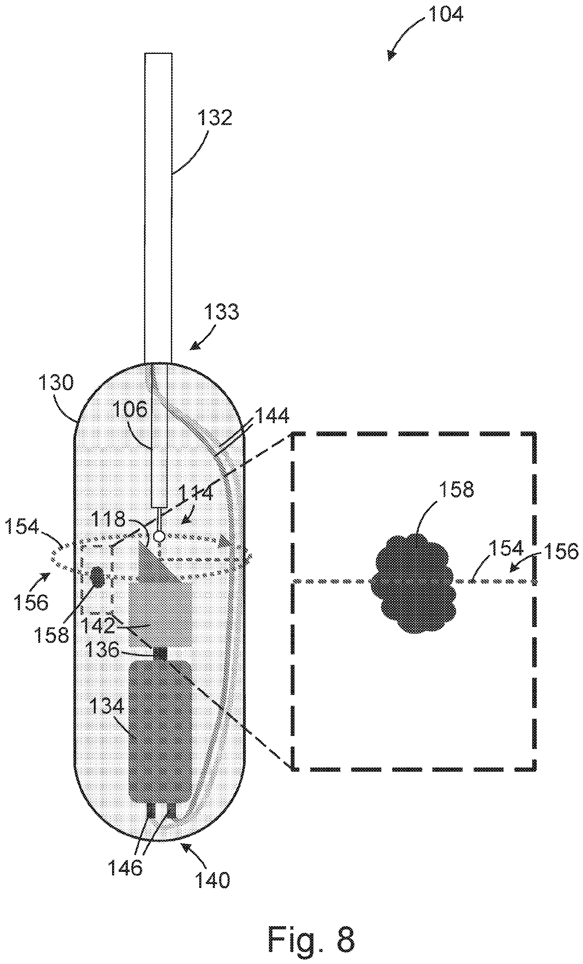

Fig. 8 illustrates another configuration for generating a feedback signal. As shown in Fig. 8, the capsule 130 may include a detection object 158 arranged on the capsule 130 and within a portion of the circumferential field of view and/or a portion of the circumferential optical path. In one example, the detection object 158 may be in the form of a light emitting material. The light emitting material may be a phosphorescent material, a fluorescent material, a quantum dot material, or a combination thereof. The light emitting material may be configured to absorb a portion of the source light reflected from reflective surface 118 and subsequently emit signal light at a wavelength different than the source light back to the reflective surface 118 and into the proximal end 114 of the optical waveguide 106. The signal light emitted from the light emitting material may be optically filtered (e.g., via a wavelength division multiplexer, a dichroic mirror) and utilized to provide a feedback control signal to the controller 126. The signal light emitted from the light emitting material may be detected as the reflective surface 118 is rotatably positioned by the motor 134 to direct the emitted signal light from the light emitting material to the proximal end 114 of the optical waveguide 106. Again, any deviation in the periodicity in the signal light emitted from the light emitting material indicates a change in the rotational speed of the motor 134 and may be utilized to provide a feedback signal to the controller 126.

In other examples, the detection object 158 may be in the form of a light reflecting material. It should be appreciated that although the configuration of Fig. 8 illustrates the wires 144 within the tethered optical imaging probe 104, the feedback signal is generated via the detection object 158. Therefore, in the example of Fig. 8, the wires 144 may not be required, and the tethered optical imaging probe 104 may alternatively be in the wireless configuration of Fig. 3.

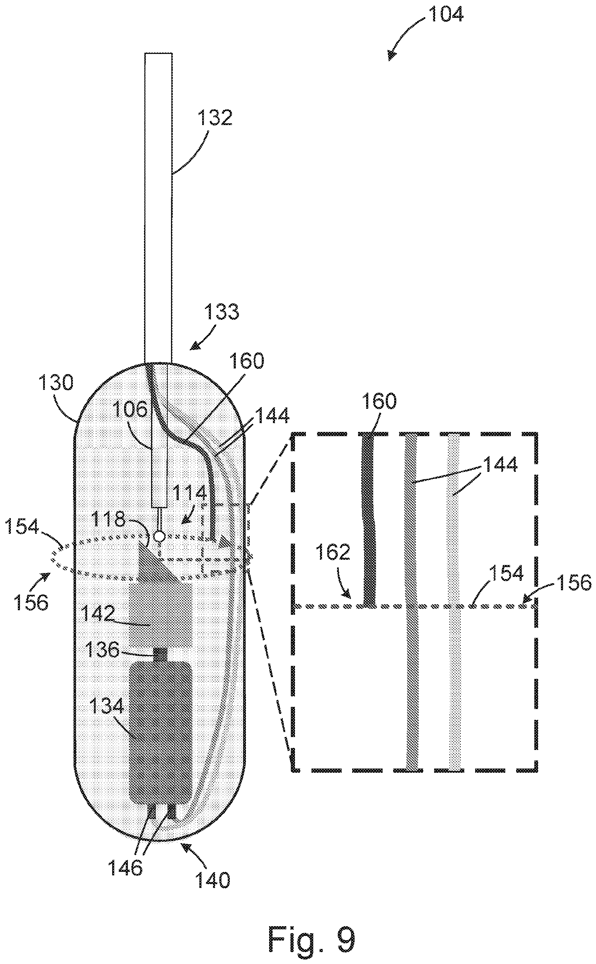

Fig. 9 illustrates another configuration for generating a feedback signal. As shown in Fig. 9, the tethered optical imaging probe 104 may include a secondary optical waveguide 160 inserted into the first end 133 of the capsule 130. The detection object may be in the form of the secondary optical waveguide 160. The secondary optical waveguide 160 is arranged receive a portion of the source light reflected from the reflective surface 118 as the reflective surface 118 is rotated by the motor 134. That is, the secondary optical waveguide 160 includes a secondary proximal end 162 arranged at least partially within the circumferential optical path. The feedback signal may be generated as the reflective surface 118 is rotatably positioned by the motor 134 to direct source light into the secondary proximal end 162 of the secondary optical waveguide 160. The feedback signal detected by the secondary optical waveguide 160 may be transmitted to a secondary distal end 164 thereof. Again, any deviation in the periodicity in the feedback signal detected by the secondary optical waveguide 160 indicates a change in the rotational speed of the motor 134 and may be utilized to provide a feedback signal to the controller 126.

It should be appreciated that although the configuration of Fig. 9 illustrates the wires 144 within the tethered optical imaging probe 104, the feedback signal is generated via the secondary optical waveguide 160. Therefore, in the example of Fig. 9, the wires 144 may not be required, and the tethered optical imaging probe 104 may alternatively be in the wireless configuration of Fig. 3.

### EXAMPLES

The following examples set forth, in detail, ways in which the optical system 100 and/or the tethered optical imaging probe 104 may be used or implemented, and will enable one of skill in the art to more readily understand the principles thereof. The following examples are presented by way of illustration.

### Motor Characterization

Exemplary operational parameters for the motor used in the Examples can be as follows: a maximum voltage applied to the motor (V_max) can be 3 V; a period of pulsed voltage applied to the motor (T) can be 1 ms; a pulse duration of pulsed voltage applied to the motor (T_d) can be 166 µs; a mean voltage applied to the motor (V_mean) can be 0.5 V; and a damping weight can be 1. 7 g. An exemplary motor used in the Examples is motor 103-100 from Precision Microdrives (available commercially from Precision Microdrives, London, United Kingdom).

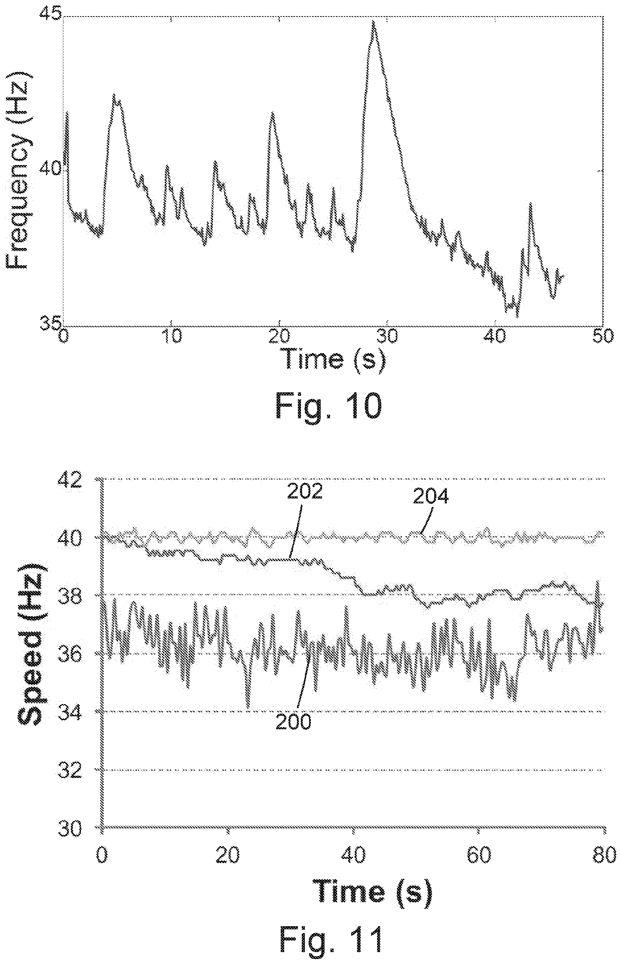

A low-cost, cell phone motor was tested to determine a speed profile of the motor as a function of time with a constant DC voltage applied thereto. As shown in Fig. 10, the motor speed (represented in frequency of rotation) substantially varies as a function of time with the constant DC voltage applied to the motor. Thus, supplying a constant DC voltage to the motor 134 is not sufficient to facilitate high precision imaging, where the rotational speed of the motor must be accurately controlled.

Next, a periodic signal was applied to the motor in an attempt to improve the accuracy and precision in the speed profile generated during operation of the motor. In addition, a feedback signal, which indicated a change in a rotational speed of the motor, was supplied to a controller to modulate the periodic signal applied to the motor. It was attempted to control the motor at a constant speed of 40 Hz. Fig 11 illustrates the results of the test with graph 200 representing a constant DC voltage applied to the motor with no feedback signal, graph line 202 representing a periodic control signal applied to the motor with no feedback signal, and graph 204 representing a periodic control signal applied to the motor with a feedback signal. As shown in Fig. 11, applying a constant DC voltage to the motor with no feedback signal Z (graph 200) resulted in an erratic speed profile that was offset from 40 Hz (i.e., averaged approximately 37 Hz). Applying a periodic control signal to the motor with no feedback loop (graph 202) improved the precision and accuracy in the speed profile as a function of time. Lastly, when a periodic signal was applied to the motor with a feedback loop (graph 204), the speed profile drastically improved in accuracy and precision, when compared to graphs 200 and 202.

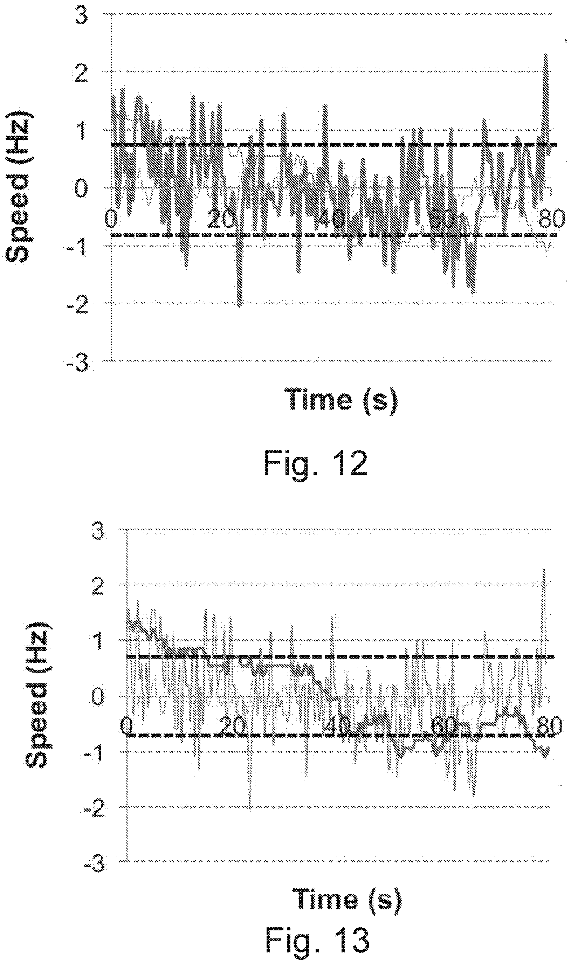

To supplement the data shown in Fig. 11, Figs. 12, 13, and 14 illustrate a deviation profile of each of graphs 200, 202, and 204, respectively. The deviation profiles were calculated by subtracting the values of the graphs 200, 202, and 204 from the corresponding means. The standard deviations defined by the derivation profiles in Figs. 12, 13, and 14 are 0.76, 0.71, and 0.17, respectively. Thus, the application of a periodic control signal to the motor and the feedback loop drastically improve the precision in the output speed profile of the motor.

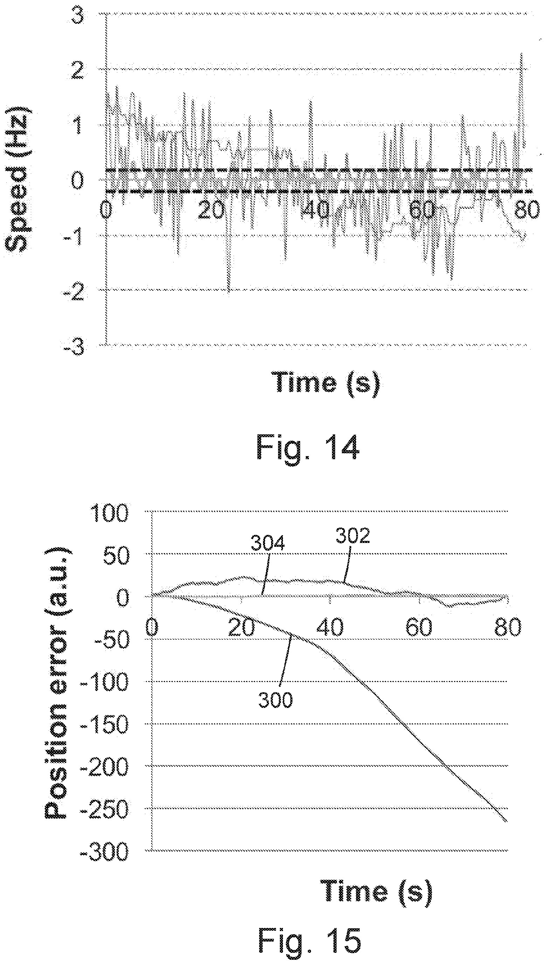

Fig. 15 illustrates a position error of the motor as a function of time for the motor with graph 300 representing a constant DC voltage applied to the motor with no feedback signal, graph 302 representing a periodic control signal applied to the motor with no feedback signal, and graph 304 representing a periodic control signal applied to the motor with a feedback signal. Position of the motor is defined as the direction in which a laser beam is directed (relative to an absolute position, like the position of wires, for example). Position error is the difference between the actual motor position and the expected motor position if the motor is spinning under ideal conditions or constant speed. As shown in Fig. 15, the position error is negligible when the periodic control signal is applied to the motor with a feedback signal.

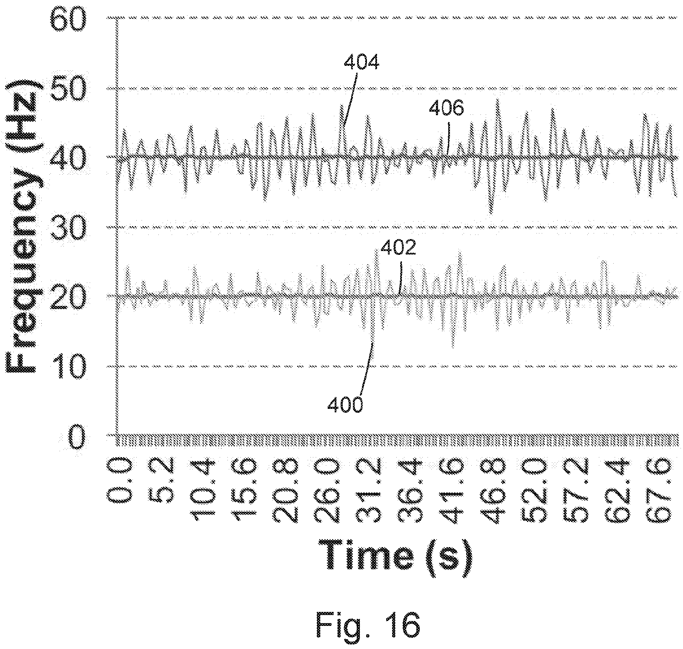

Fig. 16 illustrates the result of implementing the damping weight 142 on the feedback loop performance. The motor was controlled to 20 Hz for graphs 400 and 402 with graph 402 including the damping weight 142 and graph 400 not including the damping weight 142. The motor was controlled to 40 Hz for graphs 404 and 406 with graphs 406 including the damping weight 142 and graph 404 not including the damping weight 142. As shown in Fig. 16, the precision of the speed profile substantially improved at both 20 Hz and 40 Hz when the damping weight 142 was attached to the motor (i.e., graphs 400, 402, 404, and 406 define standard deviations of 2.5, 0.07, 3.06, and 0.166, respectively).

The invention is defined by the appended claims.

## Claims

1. A tethered optical imaging probe (104), comprising:
a motor (134);
a reflective surface (118) rotatably coupled to the motor (134);
a capsule (130) enclosing the motor (134) and the reflective surface (118), wherein the capsule (130) is dimensioned to be swallowable by a patient; and
a tether coupled to a first end of the capsule (130), the tether including an optical waveguide (106) arranged therein, wherein the optical waveguide (106) is configured to receive source light at a distal end of the optical waveguide (106) and configured to project the source light from a proximal end of the optical waveguide (106) onto the reflective surface (118) and configured to receive reflected light from the reflective surface (118) at the proximal end of the optical waveguide (106) and configured to transmit the reflected light to the distal end of the optical waveguide (106),
wherein the motor (134), the reflective surface (118), and the optical waveguide (106) are positioned to define a circumferential optical path for the reflected source light that encompasses a portion of the capsule (130), **characterized in that** a detection object in the form of a light emitting material is arranged on the portion of the capsule (130) in the circumferential optical path.

2. The tethered optical imaging probe (104) of claim 1, further comprising a light reflecting material arranged on the portion of the capsule (130).

3. The tethered optical imaging probe (104) of claim 1, wherein the light emitting material comprises a phosphorescent material, a fluorescent material, or a combination thereof.

4. The tethered optical imaging probe (104) of claim 1, wherein the light emitting material is configured to absorb a portion of the source light reflected from the reflective surface (118) and configured to subsequently emit signal light at a wavelength different than the source light back to the reflective surface (118) and into the proximal end of the optical waveguide (106).

5. The tethered optical imaging probe (104) of claim 1, wherein a signal is generated as the reflective surface (118) is rotatably positioned by the motor (134) to direct emitted light from the light emitting material to the proximal end of the optical waveguide (106) for transmission to the distal end of the optical waveguide (106).

6. The tethered optical imaging probe (104) of claim 5, wherein the signal is configured to provide feedback to a controller to enable control of a rotational speed of the motor (134).

7. The tethered optical imaging probe (104) of claim 1, wherein at least one wire extends into and along the capsule (130) to electrically couple to the motor (134).

8. The tethered optical imaging probe (104) of claim 7, wherein the probe is configured to generate a signal as the reflective surface (118) is rotatably positioned by the motor (134) to direct the source light to the at least one wire and to direct the reflected light from the at least one wire to the proximal end of the optical waveguide (106) for transmission to the distal end of the optical waveguide (106), the at least one wire being at least partially reflective to the source light and reflecting a reflected source light to the reflective surface (118).

9. The tethered optical imaging probe (104) of claim 7, wherein the at least one wire is coated with a coating configured to absorb a portion of the source light reflected from the reflective surface (118) and subsequently emit signal light at a wavelength different than the source light back to the reflective surface (118) and into the proximal end of the optical waveguide (106).

10. The tethered optical imaging probe (104) of claim 1, further comprising a secondary optical waveguide (106) inserted into the capsule (130) and configured to receive a portion of the source light reflected from the reflective surface (118).

11. The tethered optical imaging probe (104) of claim 10, wherein the probe is configured to generate a signal as the reflective surface (118) is rotatably positioned by the motor (134) to direct source light into a secondary proximal end of the secondary optical waveguide (106) for transmission to a secondary distal end of the secondary waveguide.

12. The tethered optical imaging probe (104) of claim 1, further comprising a damping weight coupled to a drive shaft of the motor (134) for rotation therewith.

13. The tethered optical imaging probe (104) of claim 1, wherein the probe is configured to supply a periodic control signal to the motor (134) to enable the rotation of the motor (134).

## Patentansprüche

1. Eine angebundene optische Bildgebungssonde (104) aufweisend:
einen Motor (134);
eine reflektierende Oberfläche (118) rotierbar gekoppelt mit dem Motor (134);
eine Kapsel (130), einschließend den Motor (134) und die reflektierende Oberfläche (118),
wobei die Kapsel (130) dimensioniert ist, um von einem Patienten schluckbar zu sein; und
ein Halteseil gekoppelt mit einem ersten Ende der Kapsel (130), wobei das Halteseil einen darin angeordneten optischen Wellenleiter (106) umfasst, wobei der optische Wellenleiter (106) konfiguriert ist, um Quellenlicht an einem distalen Ende des optischen Wellenleiters (106) zu empfangen und konfiguriert ist, um das Quellenlicht von einem proximalen Ende des optischen Wellenleiters (106) auf die reflektierende Oberfläche (118) zu projizieren und konfiguriert ist, um das reflektierte Licht von der reflektierenden Oberfläche (118) an dem proximalen Ende des optischen Wellenleiters zu empfangen und konfiguriert ist, um das reflektierte Licht zu dem distalen Ende des optischen Wellenleiters (106) zu übertragen,
wobei der Motor (134), die reflektierende Oberfläche (118), und der optische Wellenleiter (106) positioniert sind, um einen umlaufenden optischen Pfad für das reflektierende Quellenlicht zu definieren, welches einen Teil der Kapsel (130) umgibt, **dadurch gekennzeichnet** das, ein Detektionsobjekt in der Form eines Licht emittierenden Materials an dem Teil der Kapsel (130) in dem umlaufenden optischen Pfad angeordnet ist.

2. Die angebundene optische Bildgebungssonde (104) gemäß Anspruch 1, ferner aufweisend ein Licht reflektierendes Material, welches an dem Teil der Kapsel (130) angeordnet ist.

3. Die angebundene optische Bildgebungssonde (104) gemäß Anspruch 1, wobei das Licht emittierende Material ein phosphoreszierendes das Material, ein fluoreszierendes Material oder eine Kombination davon aufweist.

4. Die angebundene optische Bildgebungssonde (104) gemäß Anspruch 1, wobei das Licht emittierende Material konfiguriert ist, um einen Teil des Quellenlichts, reflektiert von der reflektierenden Oberfläche (118), zu absorbieren und konfiguriert ist um nachfolgend Signallicht mit einer anderen Wellenlänge als das Quellenlicht zurück zur reflektierenden Oberfläche (118) und in das proximale Ende des Lichtwellenleiters (106) zu emittieren.

5. Die angebundene optische Bildgebungssonde (104) gemäß Anspruch 1, wobei ein Signal generiert wird wenn die reflektierende Oberfläche (118) mittels des Motors (134) rotierbar positioniert wird, um das emittiertes Licht von dem Licht emittierenden Material zu dem proximalen Ende des optischen Wellenleiters (106) zum Übertragen zum distalen Ende des optischen Wellenleiters (106) zu richten.

6. Die angebundene optische Bildgebungssonde (104) gemäß Anspruch 5, wobei das Signal konfiguriert ist, um einem Controller eine Rückmeldung bereitzustellen, um die Steuerung einer Rotationsgeschwindigkeit des Motors (134) zu ermöglichen.

7. Die angebundene optische Bildgebungssonde (104) gemäß Anspruch 1, wobei zumindest ein Draht sich in und entlang der Kapsel (130) erstreckt, um elektrisch mit dem Motor (134) zu koppeln.

8. Die angebundene optische Bildgebungssonde (104) gemäß Anspruch 7, wobei die Sonde konfiguriert ist, um ein Signal zu generieren, wenn die reflektierende Oberfläche (118) mittels des Motors (134) rotierbar positioniert wird, um das Quellenlicht auf den zumindest einen Draht zu richten und das reflektierte Licht von dem zumindest einen Draht auf das proximale Ende des optischen Wellenleiters (106) zum Übertragen zu dem distalen Ende des optischen Wellenleiters (106) zu richten, wobei der zumindest eine Draht zumindest teilweise das Quellenlicht reflektiert und ein reflektiertes Quellenlicht auf die reflektierende Oberfläche (118) reflektiert.

9. Die angebundene optische Bildgebungssonde (104) gemäß Anspruch 7, wobei der zumindest eine Draht mit einer Beschichtung beschichtet ist, welche konfiguriert ist, um einen Teil des von der reflektierenden Oberfläche (118) reflektierten Quellenlichts zu absorbieren und nachfolgend Signallicht mit einer anderen Wellenlänge als das Quellenlicht zurück zur reflektierenden Oberfläche (118) und in das proximale Ende des optischen Wellenleiters (106) zu emittieren.

10. Die angebundene optische Bildgebungssonde (104) gemäß Anspruch 1, ferner aufweisend einen sekundären optischen Wellenleiter (106), der in die Kapsel (130) eingebracht ist und konfiguriert ist, um einen Teil des von der reflektierenden Oberfläche (118) reflektierten Quellenlichts zu empfangen.

11. Die angebundene optische Bildgebungssonde (104) gemäß Anspruch 10, wobei die Sonde konfiguriert ist, um ein Signal zu generieren, wenn die reflektierende Oberfläche (118) mittels des Motors (134) rotierbar positioniert wird, um Quellenlicht in ein sekundäres proximales Ende des sekundären optischen Wellenleiters (106) zum Übertragen zu einem sekundären distalen Ende des sekundären Wellenleiters zu richten.

12. Die angebundene optische Bildgebungssonde (104) gemäß Anspruch 1, welche ferner ein Dämpfungsgewicht aufweist, das mit einer Antriebswelle des Motors (134) gekoppelt ist, um mit diesem zu rotieren.

13. Die angebundene optische Bildgebungssonde (104) gemäß Anspruch 1, wobei die Sonde konfiguriert ist, um ein periodisches Steuersignal an den Motor (134) zu liefern, um die Rotation des Motors (134) zu ermöglichen.

## Revendications

1. Sonde d'imagerie optique attachée (104), comprenant :
un moteur (134) ;
une surface réfléchissante (118) couplée en rotation au moteur (134) ;
une capsule (130) enfermant le moteur (134) et la surface réfléchissante (118), dans laquelle la capsule (130) est dimensionnée pour pouvoir être avalée par un patient ; et
une attache couplée à une première extrémité de la capsule (130), l'attache incluant un guide d'ondes optique (106) agencé dans celle-ci, dans laquelle le guide d'ondes optique (106) est configuré pour recevoir de la lumière source à une extrémité distale du guide d'ondes optique (106) et configuré pour projeter la lumière source à partir d'une extrémité proximale du guide d'ondes optique (106) sur la surface réfléchissante (118) et configuré pour recevoir la lumière réfléchie par la surface réfléchissante (118) à l'extrémité proximale du guide d'ondes optique (106) et configuré pour transmettre la lumière réfléchie à l'extrémité distale du guide d'ondes optique (106),
dans laquelle le moteur (134), la surface réfléchissante (118) et le guide d'ondes optique (106) sont positionnés pour définir un trajet optique circonférentiel pour la lumière source réfléchie qui englobe une partie de la capsule (130), **caractérisée en ce qu'**un objet de détection sous la forme d'un matériau émetteur de lumière est agencé sur la partie de la capsule (130) dans le trajet optique circonférentiel.

2. Sonde d'imagerie optique attachée (104) selon la revendication 1, comprenant en outre un matériau réflecteur de lumière agencé sur la partie de la capsule (130).

3. Sonde d'imagerie optique attachée (104) selon la revendication 1, dans laquelle le matériau émetteur de lumière comprend un matériau phosphorescent, un matériau fluorescent ou une combinaison de ceux-ci.

4. Sonde d'imagerie optique attachée (104) selon la revendication 1, dans laquelle le matériau émetteur de lumière est configuré pour absorber une partie de la lumière source réfléchie par la surface réfléchissante (118) et configuré pour émettre ensuite en arrière une lumière-signal à une longueur d'onde différente de la lumière source vers la surface réfléchissante (118) et dans l'extrémité proximale du guide d'ondes optique (106).

5. Sonde d'imagerie optique attachée (104) selon la revendication 1, dans laquelle un signal est généré lorsque la surface réfléchissante (118) est positionnée de façon à pouvoir être entraînée en rotation par le moteur (134) pour diriger la lumière émise par le matériau émetteur de lumière vers l'extrémité proximale du guide d'ondes optique (106) en vue d'une transmission à l'extrémité distale du guide d'ondes optique (106).

6. Sonde d'imagerie optique attachée (104) selon la revendication 5, dans laquelle le signal est configuré pour fournir une rétraction à une commande pour permettre une commande d'une vitesse de rotation du moteur (134).

7. Sonde d'imagerie optique attachée (104) selon la revendication 1, dans laquelle au moins un fil s'étend dans et le long de la capsule (130) pour être électriquement couplé au moteur (134).

8. Sonde d'imagerie optique attachée (104) selon la revendication 7, dans laquelle la sonde est configurée pour générer un signal lorsque la surface réfléchissante (118) est positionnée de façon à pouvoir être entraînée en rotation par le moteur (134) pour diriger la lumière source vers le au moins un fil et pour diriger la lumière réfléchie par le au moins un fil vers l'extrémité proximale du guide d'ondes optique (106) en vue d'une transmission à l'extrémité distale du guide d'ondes optique (106), le au moins un fil étant au moins partiellement réfléchissant à la lumière source et réfléchissant une lumière source réfléchie par la surface réfléchissante (118).

9. Sonde d'imagerie optique attachée (104) selon la revendication 7, dans laquelle le au moins un fil est revêtu d'un revêtement configuré pour absorber une partie de la lumière source réfléchie par la surface réfléchissante (118) et émettre ensuite en arrière une lumière-signal à une longueur d'onde différente de la lumière source vers la surface réfléchissante (118) et dans l'extrémité proximale du guide d'ondes optique (106).

10. Sonde d'imagerie optique attachée (104) selon la revendication 1, comprenant en outre un guide d'ondes optique secondaire (106) inséré dans la capsule (130) et configuré pour recevoir une partie de la lumière source réfléchie par la surface réfléchissante (118).

11. Sonde d'imagerie optique attachée (104) selon la revendication 10, dans laquelle la sonde est configurée pour générer un signal lorsque la surface réfléchissante (118) est positionnée de façon à pouvoir être entraînée en rotation par le moteur (134) pour diriger la lumière source dans une extrémité proximale secondaire du guide d'ondes optique secondaire (106) en vue d'une transmission à une extrémité distale secondaire du guide d'ondes secondaire.

12. Sonde d'imagerie optique attachée (104) selon la revendication 1, comprenant en outre un poids d'amortissement couplé à un arbre d'entraînement du moteur (134) en vue d'une rotation avec celui-ci.

13. Sonde d'imagerie optique attachée (104) selon la revendication 1, dans laquelle la sonde est configurée pour fournir un signal de commande périodique au moteur (134) pour permettre la rotation du moteur (134).
